# EUROPÄISCHE PATENTSCHRIFT

(11) **EP 3 315 161 B1**
(45) Veröffentlichungstag und Bekanntmachung des Hinweises auf die Patenterteilung: **04.10.2023**
(21) Anmeldenummer: 17197570.9
(22) Anmeldetag: 20.10.2017
(51) Int. Cl.: A61M 25/00, A61M 27/00, A61M 25/01

(54) **KATHETERSYSTEM ZUR INTERMITTIERENDEN KATHETERISIERUNG**
CATHETER SYSTEM FOR INTERMITTENT CATHETERIZATION
SYSTÈME DE CATHÉTER POUR CATHÉTÉRISME INTERMITTENT

(30) Priorität: 25.10.2016 DE 102016120294
(43) Veröffentlichungstag der Anmeldung: 02.05.2018
(73) Patentinhaber: UROMED KURT DREWS KG, 22113 Oststeinbek (DE)
(72) Erfinder: Le Cerf, Nils, 22609 Hamburg (DE)
(74) Vertreter: Eisenführ Speiser

(56) Entgegenhaltungen:
- US-A- 4 170 996
- US-A1- 2006 025 753
- US-A1- 2009 071 851
- US-A1- 2015 133 898

## Beschreibung

### Hintergrund

Die Erfindung betrifft ein Kathetersystem zur intermittierenden Katheterisierung, d. h. Ableitung von Urin, der im Oberbegriff des Anspruchs 1 genannten Art, insbesondere ein Kathetersystem zur intermittierenden Selbst-Katheterisierung.

Bei Patienten mit Blasenfunktionsstörungen kann es notwendig sein, die Urinableitung regelmäßig mit Hilfe eines Katheters durchzuführen. Neurogene Blasenfunktionsstörungen treten z. B. bei Patienten mit Querschnittslähmung z. B. nach einer Rückenmarksverletzung auf. Das zur Blasenentleerung notwendige Kontrahieren der Blasenmuskulatur und der Blasenschließmuskel können bei solchen Patienten oft gar nicht mehr oder nur noch zeitweise bewusst gesteuert werden. Die Patienten sind daher auf Katheter angewiesen, mit deren Hilfe sie intermittierend, z. B. etwa alle 3 Stunden, ihre Harnblase entleeren können (Intermittierender Selbstkatheterismus, ISK).

Um in den Alltag der Patienten komfortabel integriert werden zu können, sollten die Katheter steril sein und vorzugsweise eine möglichst geringe Keimverschleppung aus der Harnröhre in die Harnblase sicherstellen und gleichzeitig auch von motorisch eingeschränkten Patienten einfach gehandhabt werden können.

Keime können beim Katheterisierungsvorgang während des Vorschiebens des Katheters in die Blase gelangen, insbesondere da wesentliche Teile der Keime sich in den ersten 1,5 cm des Harnröhreneingangs befinden. Von dort können die Keime in den Rest der Harnröhre und die Harnblase gelangen. Um dies zu vermeiden, werden im Stand der Technik im ISK-Bereich z. B. Katheter mit Einführelementen eingesetzt, die eine Vorlaufspitze umfassen, die in diesen Anfangsbereich der Harnröhre eingeschoben werden kann und den keimbelasteten Bereich somit überbrückt. Anschließend wird der Kathetertubus durch eine flexibel verschließbare Öffnung in der Vorlaufspitze in die Harnröhre und bis in die Blase eingeschoben. Auch die Gefahr eines Herausrutschens des Katheters kann durch das Einführelement reduziert werden, was Patienten mit motorischen Einschränkungen die intermittierende Selbst-Ableitung von Urin deutlich erleichtert. Die Einführelemente stellen somit eine deutliche Verbesserung der ISK Katheter dar. Beispielhafte Einführelemente sind z. B. in US 2014/0276661 A1 beschrieben.

Trotz dieser Verbesserungen der Sterilität fällt es gerade Patienten mit motorischen Einschränkungen, wie z. B. Verkrampfungen der Muskulatur, oft schwer, die ISK Katheter aus ihrer sterilen Umverpackung zu entnehmen und einzuführen, ohne sie einer Keimbelastung auszusetzen. Es wäre daher wünschenswert, Katheter und Katheterverpackungen derart weiterzuentwickeln, dass gute Voraussetzungen für die Bewahrung der Kathetersterilität auch bei Benutzung durch motorisch eingeschränkte Patienten gewährleistet werden.

Darüber hinaus haben die verpackten Katheter meist ein für den Transport sehr unpraktisches Format. Die Katheter für männliche Patienten weisen z. B. in der Regel eine unhandliche Länge von etwa 41 cm auf. Diese Katheter sind in Standardsituationen häufig schwer zu transportieren, z. B. wenn der Patient ohne eine größere Tasche unterwegs ist. Es wäre daher ebenfalls wünschenswert, die Katheterverpackungen und Katheter derart weiterzuentwickeln, dass der Transport erleichtert wird.

US 2015/0202405 A1 beschreibt ein Katheterverpackungssystem, das aus einem spiralförmigen Stauraum für einen Katheter besteht. Der Katheter kann so in einer aufgerollten Anordnung transportiert werden. In DE 20 2005 009 946 U1 ist ein Kathetersystem mit einer flächigen Verpackung mit einem Katheteraufnahmevolumen, in dem der Blasenkatheter angeordnet ist, beschrieben. Die Verpackung weist ein Urinbeutelvolumen auf, in dem der abgeleitete Urin aufgefangen werden kann. Beide Systeme weisen zwar ein besseres Transportformat als Standardverpackungen auf, sie sind aber dennoch für motorisch eingeschränkte Patienten zu diffizil in der Handhabung. Gute Sterilbedingungen bei der Einführung der Katheter sind somit nicht gewährleistet.

US 2015/133898 A1 offenbart eine Katheterverpackung mit einem Grundkörper und einem Deckel sowie einer Öffnung im Bereich des proximalen Endes des Katheters.

Aufgabe der vorliegenden Erfindung ist es daher, einen gattungsgemäßen Katheter bereitzustellen, der zur intermittierenden Selbst-Ableitung von Urin durch eine motorisch eingeschränkte Person geeignet ist und dieser Person eine schnelle, zuverlässige und hygienische Handhabung ermöglicht und darüber hinaus unauffällig und mit geringem Transportvolumen zu transportieren ist.

### Beschreibung der Erfindung

Gelöst wird die Aufgabe durch Kathetersysteme zur intermittierenden Katheterisierung mit den Merkmalen des Anspruchs 1.

Die Erfindung betrifft somit ein Kathetersystem zur intermittierenden Katheterisierung, insbesondere zur intermittierenden Selbst-Katheterisierung, umfassend eine schlauchförmige Verpackung und einen die Verpackung durchlaufenden Katheter, dadurch gekennzeichnet, dass die schlauchförmige Verpackung ringförmig geschlossen ist.

Wie zuvor beschrieben, sind Kathetersysteme zur intermittierenden (Selbst-) Katheterisierung für menschliche Individuen bekannt. Gegenüber diesen bekannten ISK Kathetersystemen bringen die erfindungsgemäßen Kathetersysteme jedoch den Vorteil mit sich, dass sie unauffällig transportiert und auch von motorisch eingeschränkten Patienten leicht und ohne wesentliche Einschränkungen der Sterilität geöffnet und eingeführt werden können. Die Optimierung des Transportvolumens und der Transportform wirkt sich insbesondere bei den sehr langen Kathetern für männliche Patienten positiv aus. Das Infektionsrisiko ist bei Benutzung der erfindungsgemäßen Kathetersysteme gegenüber herkömmlichen Systemen minimiert.

Ein unauffälliger Transport wird insbesondere durch die Flexibilität der Verpackung sichergestellt. Die schlauchförmige Verpackung ist ringförmig geschlossen. Dieser Ring lässt sich zu einer Ellipse zusammendrücken, insbesondere zu einer Ellipse mit einem sehr geringen Abstand zwischen ihren Nebenscheitelpunkten. Vorzugsweise lässt sich die ringförmig geschlossene Verpackung so weit zusammendrücken, dass die Nebenscheitelpunkte der zwischenzeitlich entstandenen Ellipse sich berühren. Dies ist ein besonderer Vorteil gegenüber bisher bestehenden Verpackungssystemen, die häufig starr sind und daher keinen flexiblen Transport ermöglichen.

"Ringförmig geschlossen" im Sinne der Erfindung bedeutet, dass das distale Ende der schlauchförmigen Verpackung angrenzend mit dem proximalen Ende der schlauchförmigen Verpackung verbunden ist. Die Begriffe "distales" und "proximales" Ende der Verpackung bezeichnen in diesem Zusammenhang die Richtung der Enden im geöffneten Verpackungszustand, wie an anderer Stelle hierin näher erläutert.

Der Begriff intermittierende (Selbst-)Katheterisierung bezeichnet die intermittierende (d. h. zeitweilig aussetzende) (Selbst-)Ableitung von Urin mittels eines Katheters. Ein Katheter im Sinne der Erfindung ist somit ein Harnblasenkatheter bzw. Blasenkatheter, d. h. ein Katheter zur Ableitung von Urin. Als intermittierende Selbst-Katheterisierung wird insbesondere die vom Patienten selber durchgeführte Eigenkatheterisierung bezeichnet, die regelmäßig wiederholt wird. Die erfindungsgemäßen Katheter können selbstverständlich auch für die Katheterisierung am Patienten durch medizinisches Fachpersonal eingesetzt werden. Die Verwendung für die Selbst-Katheterisierung ist bevorzugt. Die zeitlichen Abstände der Wiederholung (die Frequenz) sind individuell und je nach Beschwerden, der Trinkmenge und den vom Patienten eingenommenen Medikamenten anzupassen. In der Regel wird die Katheterisierung vier bis sechs Mal am Tag durchgeführt.

Aufgrund der anatomischen Unterschiede zwischen den Urogenitalsystemen von Mann und Frau sind Katheter in der Regel nur für Personen eines Geschlechts geeignet. Katheter für Männer sind deutlich länger als die für Frauen. Die erfindungsgemäßen Katheter können speziell an das Urogenitalsystem des Mannes oder einer Frau angepasst werden und somit dann für die Anwendung durch einen männlichen bzw. einen weiblichen ISK Patienten bzw. ein männliches bzw. weibliches, menschliches Individuum besonders geeignet sein. Die erfindungsgemäßen Katheter können für die Verwendung durch einen Mann oder eine Frau geeignet sein. Bevorzugt sind die Katheter für die Verwendung durch einen Mann geeignet.

Das Kathetersystem umfasst eine schlauchförmige Verpackung. Solche schlauchförmigen Verpackungen sind Fachleuten im Prinzip bekannt und dienen bislang entweder dem Einschluss einer Menge an Gleitmittel um den Kathetertubus herum oder der umschließenden, sterilen Verpackung des gesamten Katheters. Im Gegensatz zu den Meisten herkömmlichen schlauchförmigen Verpackungen, ist die erfindungsgemäße Verpackung sowohl das Reservoir für ein Gleitmittel als auch eine Sterilverpackung für den gesamten Katheter. Die schlauchförmige Verpackung stellt somit eine Sterilbarriere dar. Erfindungsgemäß wird daher nur eine Umhüllung des Kathetertubus und Katheters benötigt. Hierdurch wird die Handhabung auch durch motorisch eingeschränkte Patienten erleichtert. Darüber hinaus unterscheidet sich die Verpackung auch dadurch von Standardumhüllungen, dass die Verpackung, wie an anderer Stelle hierin beschrieben, ringförmig geschlossen ist.

"Schlauchförmig" bedeutet, dass die Verpackung in ihrer Form einem Schlauch ähnelt. Mit anderen Worten weist die Verpackung, mindestens auf ihrer Innenseite, im Wesentlichen die Form eines flexiblen, länglichen Tubus, d.h. eines flexiblen, länglichen Hohlzylinders, auf. Die Verpackung kann z. B. aus einem geeigneten Material als Schlauch extrudiert werden. Alternativ kann die Verpackung aber auch durch das Verschweißen oder Verkleben zweier länglicher Folien an ihren langen Seiten oder durch das Verschweißen oder Verkleben der beiden langen Seiten einer länglichen Folie in eine Schlauchform gebracht werden. Es versteht sich von selbst, dass durch diese Verfahrensschritte die Tubus-Form der schlauchförmigen Verpackung auf der Außen- und/oder Innenseite der Verpackung Unregelmäßigkeiten, wie z. B. einen Grat oder mehrere Grate, aufweisen kann.

Weiter alternativ kann die ringförmig geschlossene Verpackung auch hergestellt werden, indem zwei flächige Folien übereinander gelegt werden, die jeweils einen Durchmesser aufweisen, der einen Durchmesser des geschlossenen Verpackungsrings übersteigt. Anschließend werden zwei kreisförmige, konzentrische Schweisslinien mit dem Außen- und dem Innendurchmesser des Verpackungsrings auf die beiden Folien aufgebracht. Überstehende Ränder können anschließend innerhalb und außerhalb der Schweißnähte entfernt werden, so dass ein ringförmiger Schlauch entsteht.

Die Verpackung ist derart flexibel, dass sie in eine lineare, Kreis- oder Ellipsenform geformt werden kann. Darüber hinaus lässt sich die Verpackung, wie derzeit bekannte Kathetertubusumhüllungen, während des Einführens des Kathetertubus in die Harnröhre in Längsrichtung des Kathetertubus zusammenstauchen. Diese Zusammenstauchung bleibt während des Einführvorganges bestehen.

Gleichzeitig hat der Kathetertubus ein derartiges Formgedächtnis, dass er und die Verpackung die Kreisform wieder zugunsten einer im Wesentlichen gestreckten Form aufgeben, wenn die ringförmig geschlossene Verpackung geöffnet und damit der Ringschluss unterbrochen ist.

Geeignete Materialien zur Herstellung der Verpackung sind im Stand der Technik bekannt. Die Verpackung ist z. B. aus einem Material, das geeignet ist, als eine Sterilbarriere zu wirken und gleichzeitig die oben beschriebenen Flexibilitätseigenschaften aufweist. Bevorzugt ist die Verpackung aus einem Material, das ausgewählt ist aus der Gruppe bestehend aus Kunststoffen, Polyamid (PA), Nylon (PA6), Polyethylen (PE), Polyethylenterephtalat (PET) und Verbundstoffen daraus.

Die schlauchförmige Verpackung umschließt im geschlossenen Zustand den gesamten Katheter, insbesondere inklusive der distalen und proximalen Enden des Katheters. Somit können neben dem Kathetertubus z. B. auch ein optionaler Auslass des Katheters an dessen proximalen Ende und/oder ein optionales Einführelement an dem distalen Ende im geschlossenen Verpackungszustand von der Verpackung umschlossen werden. Dies stellt sicher, dass neben der schlauchförmigen und ringförmig geschlossenen Verpackung keine weitere sterile Umverpackung erforderlich ist. Anders gesagt, ist die schlauchförmige Verpackung des erfindungsgemäßen Kathetersystems daher bevorzugt von keiner weiteren Verpackung umgeben, insbesondere nicht von einer weiteren Sterilverpackung.

Darüber hinaus umfasst das Kathetersystem einen die Verpackung durchlaufenden Katheter. Geeignete Katheter für die Intermittierende (Selbst-)Katheterisierung sind Fachleuten bekannt und an anderer Stelle hierin näher beschrieben. Der Katheter durchläuft die schlauchförmige Verpackung in Längsrichtung. Anders gesagt liegt der Katheter im Inneren der schlauchförmigen Verpackung, d. h. im Inneren des Tubus.

Das Verpackungsinnere, d. h. der Kanal im Inneren der schlauchförmigen Verpackung, hat bevorzugt einen deutlich größeren Durchmesser als der Kathetertubus. Auf diese Weise wird einerseits sichergestellt, dass der Kanal ausreichend Platz für größere Elemente des Katheters, wie z. B. ein optional vorhandenes Einführelement, bereitstellt, und andererseits genügend Platz für ein Gleitmittel im Verpackungsinneren zur Verfügung steht. Darüber hinaus ist die schlauchförmige Verpackung somit in der Lage, sich entsprechend stark zusammenschieben zu lassen, wenn der Kathetertubus eingeführt wird. Das Verpackungsinnere weist orthogonal zur Längs- bzw. Umfangsrichtung der Verpackung einen Durchmesser von z. B. 1 cm bis 3 cm, vorzugsweise 1,5 cm bis 2,5 cm auf. Das Verpackungsinnere kann auf seiner gesamten Länge im Wesentlichen den gleichen Durchmesser aufweisen. Alternativ kann die schlauchförmige Verpackung auch in unterschiedlichen Abschnitten verschiedene Durchmesser aufweisen. So kann die Verpackung z. B. einen größeren Durchmesser in einem Abschnitt aufweisen, der ein Einführelement aufweist, und einen kleineren Durchmesser in einem Abschnitt aufweisen, der von dem Kathetertubus durchlaufen wird. In bevorzugten Ausführungsformen weisen alle Abschnitte des Verpackungsinneren orthogonal zur Längsrichtung einen Durchmesser von z. B. 1 cm bis 3 cm, vorzugsweise 1,5 cm bis 2,5 cm auf. Der hier erwähnte Durchmesser bezeichnet jeweils den Durchmesser im aufgespannten und nicht im flachgedrückten Zustand.

Katheter und Verpackung werden in der Regel im Wesentlichen die gleiche Länge aufweisen. Die Verpackung kann allerdings auch kürzer sein als der Katheter, wenn das distale und proximale Ende des Katheters, z. B. das optional vorhandene Einführelement und der optional vorhandene Auslass, im geschlossenen Zustand der Verpackung neben- oder ineinander liegen, oder länger sein als der Katheter, wenn das distale und das proximale Ende des Katheters, z. B. das optional vorhandene Einführelement und der optional vorhandene Auslass, im ringförmig geschlossenen Zustand der Verpackung in Längs- bzw. Umfangsrichtung der Verpackung voneinander beabstandet sind.

Die schlauchförmige Verpackung des erfindungsgemäßen Kathetersystems ist ringförmig geschlossen. Dies bedeutet, dass das distale und das proximale Ende der Verpackung im geschlossenen Zustand der Verpackung miteinander verbunden sind. Im geschlossenen Zustand weist die Verpackung somit eine ringförmig geschlossene Schlauchform auf.

Das distale Ende der Verpackung ist dasjenige Ende der Verpackung, das nach dem Öffnen der Verpackung durch den Benutzer das distale Ende des Katheters umschließt. Zur Herstellung des Ringschlusses können das distale und das proximale Ende der Verpackung miteinander verbunden werden, z. B. durch Verschweißen oder Verkleben der beiden Enden oder Endbereiche. Alternativ kann der Ringschluss aber auch an anderer Stelle des durch die Verpackung gebildeten Rings hergestellt sein oder durch andere z. B. an anderer Stelle hierin beschriebene Verfahren erzeugt worden sein. In diesem Falle können sich die Enden der Verpackung nach dem Öffnen durch den Benutzer von den Enden der Verpackung vor dem Herstellen des Ringschlusses unterscheiden, d. h. sie nehmen eine andere Position innerhalb der Verpackung ein.

Der Ringschluss kann durch Verschweißen oder Verkleben des distalen Endbereichs der Verpackung mit dem proximalen Endbereich oder, wie oben beschrieben, anderer Bereiche der Verpackung hergestellt sein, insbesondere durch Verschweißen mittels Laserschweißen oder Utraschallschweißen. Dem Fachmann sind geeignete Klebstoffe und Klebeverfahren zur Herstellung des Ringschlusses bekannt. So kann zum Verkleben z. B. die Verpackung z. B. flachgedrückt und an den Katheter angedrückt werden, so dass nach Verkleben eine geschlossene Struktur entsteht. Geeignete Klebstoffe umfassen im Wesentlichen alle in der Medizintechnik geläufigen und biokompatiblen Klebstoffe zur Klebung der an anderer Stelle hierin erwähnten Verpackungsmaterialien.

Der distale Endbereich der Verpackung bezeichnet den Bereich der Verpackung, der sich nach dem Öffnen der Verpackung um das distale Ende und den distalen Endbereich des Katheters herum erstreckt. Das distale Ende des Katheters ist das Ende, das in den Penis eingeführt wird. Das proximale Ende des Katheters ist das Ende, aus dem der Urin nach dem Einführen des Kathetertubus in die Harnblase aus dem Katheter heraus ablaufen kann, d. h. es ist das Ende, das in der Regel bei Benutzung des Katheters am weitesten von der Körpermitte des Patienten entfernt sein wird. Der "Endbereich" umfasst das Ende und ist darüber hinaus jeweils der Bereich, der in der unmittelbaren Nähe des jeweiligen Endes liegt, z. B. in demselben Drittel, vorzugsweise in demselben Viertel des jeweiligen Objekts wie das jeweilige Ende (z. B. im selben Drittel des Katheters oder der Verpackung). Der Endbereich kann z. B. 5 cm oder weniger, bevorzugt 3 cm oder weniger, stärker bevorzugt 1 cm oder weniger von dem betreffenden Ende (z. B. dem distalen oder proximalen Ende) entfernt sein.

Der erfindungsgemäße Katheter umfasst einen Kathetertubus. Der Kathetertubus ist röhren- bzw. schlauchartig ausgebildet. Durch den Kathetertubus hindurch kann Urin abfließen bzw. abgeleitet werden. Geeignete Kathetertuben sind dem Fachmann bekannt. Die Kathetertuben weisen eine Länge auf, die das Erreichen der Harnblase und insbesondere das Überwinden des Blasenschließmuskels ermöglicht. Bevorzugt weist der Kathetertubus daher eine Länge von 25 cm oder mehr, vorzugsweise von 30 cm oder mehr, stärker bevorzugt von 35 cm oder mehr, z. B. von etwa 41 cm, auf. Anders gesagt weist der Kathetertubus eine Länge von 25 cm bis 60 cm, bevorzugt von 30 cm bis 50 cm auf. Solche Katheter sind aufgrund ihrer Länge insbesondere für Männer geeignet. In alternativen Ausführungen, die insbesondere für Frauen geeignet sind, weist der Kathetertubus eine Länge von 5 cm bis 25 cm, bevorzugt von 10 cm bis 20 cm auf. Für Kinder oder Jugendliche können diese Längen selbstverständlich entsprechend angepasst werden. Kathetertuben für männliche Kinder können z. B. eine Länge von 15 cm oder mehr, bevorzugt von 18 cm oder mehr, wie etwa 22 cm aufweisen. Anders gesagt weisen Kathetertuben für männliche Kinder z. B. eine Länge von 15 cm bis 29 cm, bevorzugt von 18 cm bis 26 cm auf. Kathetertuben für weibliche Kinder können entsprechend kürzere Längen aufweisen.

Der Kathetertubus weist ferner eine Steifigkeit auf, die sicherstellt, dass der Kathetertubus nicht durch den Blasenschließmuskel zusammengedrückt und damit verschlossen wird. Entsprechende Kathetertuben sind dem Fachmann bekannt.

Der Außendurchmesser des Kathetertubus kann 6 bis 20 Charrière, bevorzugt 14 bis 18 Charrière betragen. 3 Charrière entsprechen 1 mm. Kathetertuben für männliche Kinder weisen bevorzugt einen Außendurchmesser von 6 bis 10 Charrière auf.

Geeignete Materialien und (z. B. hydrophile) Beschichtungen für Kathetertuben sind dem Fachmann bekannt. Erfindungsgemäß kann jegliches in diesem Zusammenhang geeignete Material verwendet werden.

Das distale Ende des Kathetertubus ist stets, insbesondere auch wenn der Katheter kein Einführelement an seinem distalen Ende umfasst, atraumatisch geformt. So werden Verletzungen und Gewebsreizungen weitest möglich verhindert. Darüber hinaus kann die Verletzungsgefahr weiter verringert werden und die Handhabung des Katheters vereinfacht werden, indem ein unterstützendes Einführelement am distalen Katheterende vorgesehen wird. Der Katheter kann somit ferner ein Einführelement an seinem distalen Ende und/oder (zur weiteren Vereinfachung der Handhabung) einen Auslass an seinem proximalen Ende umfassen. Darüber hinaus kann der Katheter weitere geeignete Elemente umfassen.

So umfasst der Katheter an seinem distalen Ende in einer Ausführungsform ein Einführelement. Geeignete Einführelemente sind dem Fachmann ebenfalls bekannt und werden in jüngerer Zeit bereits für Katheter eingesetzt.

Das Einführelement kann an seinem distalen Ende einen konusförmigen Teil mit einer abgerundeten Spitze aufweisen (die Vorlaufspitze). Die Vorlaufspitze weist eine verschließbare Öffnung (eine Tubusdurchführung) auf, die das Durchschieben des Kathetertubus ermöglicht und gleichzeitig den Eintrag von Keimen im verschlossenen Zustand minimiert. Die Vorlaufspitze kann in die Harnröhre eingeführt werden und weist einen diesem Zweck entsprechenden Durchmesser und Länge auf. Die Tubusdurchführung kann z. B. aus einem oder mehreren, einander kreuzenden Schlitzen zusammengesetzt sein, d.h. z.B. kreuz- oder sternförmig ausgebildet sein.

Proximal schließt sich an den konusförmigen Teil ein diesen Teil kreisförmig umlaufender Kragen an. Der Kragen weist einen Außendurchmesser auf, der groß genug ist, um das Einführen des Einführelements zu stoppen, d. h. der größer ist (z. B. mindestens doppelt so groß) als der Durchmesser der Harnröhre.

Weiter proximal schließt sich an den Kragen ein länglicher Spitzenhalter an, der mindestens abschnittsweise röhrenartig ausgebildet ist und den Kathetertubus umgibt. Der Spitzenhalter dient der Stabilisierung des Tubusteils und des Einführelements sowie der leichteren Handhabbarkeit durch den Anwender. Der Spitzenhalter kann z. B. eine Hohlzylinder- oder Trichterform aufweisen, wobei die breitere Trichterseite sich proximal an den Kragen anschließt. In einer Ausführungsform ist am Spitzenhalter die Verpackung angebracht, z. B. mit ihm verklebt. Dieses Anbringen der Verpackung am Spitzenhalter verhindert ein Verrutschen der Verpackung während des Einführens des Kathetertubus in die Harnröhre.

Das Einführelement kann einstückig ausgebildet sein oder aus verschiedenen Elementen zusammengesetzt, z. B. geklebt, geklemmt oder gesteckt sein. So können z. B. Spitzenhalter, Kragen und/oder Vorlaufspitze jeweils einzelne Teile sein. Entsprechend ist angedacht, die unterschiedlichen Teile eines Einführelements aus unterschiedlichen Materialien auszubilden. So kann die Vorlaufspitze z. B. aus einem anderen Material hergestellt sein als der Spitzenhalter und der Kragen. Derartige Ausführungen sind dem Fachmann bekannt. Alternativ können Spitzenhalter, Kragen und/oder Vorlaufspitze aber auch aus demselben Material hergestellt sein, z. B. wenn das Einführelement einstückig ausgebildet ist.

Das Einführelement bzw. der Spitzenhalter, der Kragen und/oder die Vorlaufspitze bestehen vorzugsweise aus einem Kunststoff, wie z. B. Silikon. Vorzugsweise besteht das Einführelement aus Spitzenhalter, Kragen und Vorlaufspitze.

Im unbenutzten Zustand ist das distale Ende des Kathetertubus in die Vorlaufspitze des Einführelements eingeführt. Die Vorlaufspitze kann dann in die Harnröhre eingeführt werden. Anschließend wird das distale Ende des Kathetertubus durch das Einführelement, und insbesondere die Vorlaufspitze, sowie durch die Harnröhre (Urethra) bis in die Harnblase eingeführt. Das Einführen und Anlegen des erfindungsgemäßen Katheters geschieht bevorzugt nach Zurückziehen der Vorhaut, um eine stabile Halterung des Katheters zu gewährleisten.

Der Katheter kann ferner an seinem proximalen Ende einen Auslass umfassen. Der Auslass kann trichter- oder kegelförmig ausgebildet sein. Dabei ist dasjenige Ende mit einem größeren Durchmesser in der Regel proximal des Kathetertubus angeordnet. Der Auslass ist geeignet, um den abgeleiteten Urin möglichst zielgerichtet in ein weiteres Behältnis abzulassen. Alternativ oder zusätzlich zur Trichterform kann der Auslass auch ein oder mehrere Verbindungselemente zu einer Auffangeinrichtung aufweisen, mit denen der Auslass mit der Auffangeinrichtung, z. B. einem Urinbeutel, verbunden werden kann.

Das distale Ende des Katheters bzw. das Einführelement kann in der Verpackung neben, vor oder innerhalb des proximalen Endes des Katheters bzw. des Auslasses liegen. "Neben" und "vor" beziehen sich jeweils auf die Positionierung in Längs- bzw. Umfangsrichtung der ringförmig geschlossenen Verpackung. Liegt das distale Ende des Katheters bzw. das Einführelement vor dem proximalen Ende des Katheters bzw. dem Auslass, so ist das distale Ende des Katheters bzw. des Einführelements am dichtesten an dem proximalen Ende des Katheters bzw. des Auslasses. Liegt das distale Ende des Katheters bzw. das Einführelement innerhalb des proximalen Endes des Katheters bzw. des Auslasses, so ist das distale Ende bzw. der distale Endbereich des Katheters bzw. des Einführelements, z. B. die Vorlaufspitze bis zum Kragen, in das proximale Ende des Katheters oder den Auslass eingeschoben. Auf diese Weise kann die Umfangslänge der Verpackung klein gehalten und das distale Ende des Katheters bzw. das Einführelement vor Beschädigungen bewahrt werden.

Darüber hinaus könnte das distale Ende bzw. der distale Endbereich des Katheters bzw. des Einführelements, z. B. die Vorlaufspitze, auch mechanisch mit dem proximalen Ende des Katheters oder dem Auslass, z. B. durch einen Verriegelungsmechanismus, z. B. mit dem Inneren des Auslasses, verbunden sein. Erfindungsgemäß wird eine solche mechanische Verbindung oder Verriegelungsmechanismus jedoch das Öffnen der Katheterverpackung und das anschließende Einführen des Katheters für den motorisch eingeschränkten Patienten nicht komplizierter bzw. schwieriger machen. Dies bedeutet, dass eine mechanische Verbindung bzw. ein Verriegelungsmechanismus im Sinne der Erfindung z. B. keine zusätzliche Drehbewegung oder dergleichen erforderlich macht. Stattdessen kann die Verpackung durch einfaches Aufreißen geöffnet werden. Dieses Aufreißen wird vorzugsweise durch Zugausübung auf die Sollreißstelle von beiden Seiten in im Wesentlichen entgegengesetzte Richtungen bewirkt. Das Aufreißen der Verpackung trennt dabei auch das distale Ende des Katheters von dem proximalen Ende des Katheters. So wird z. B. durch das Aufreißen der Verpackung die Vorlaufspitze aus dem proximalen Ende des Katheters bzw. dem Auslass herausgezogen.

Wie oben beschrieben, kann die Verpackung vorzugsweise eine Sterilbarriere sein. Dies bedeutet, dass die Verpackung mindestens während der normalen Lebens- bzw. Lagerdauer des Kathetersystems keimdicht ist und daher sicherstellt, dass das sterile Verpackungsinnere nicht durch Keime verunreinigt wird. So wird eine Keimbelastung des Katheters vor dem Auspacken durch den Benutzer im Wesentlichen ausgeschlossen und das Infektionsrisiko deutlich reduziert.

Zwischen der Innenseite der Verpackung und dem Kathetertubus kann wie zuvor beschrieben ein Gleitmittel eingebracht sein. Für die intermittierende Katheterisierung geeignete Gleitmittel und Gleitmittelmengen sind im Stand der Technik bekannt und erprobt.

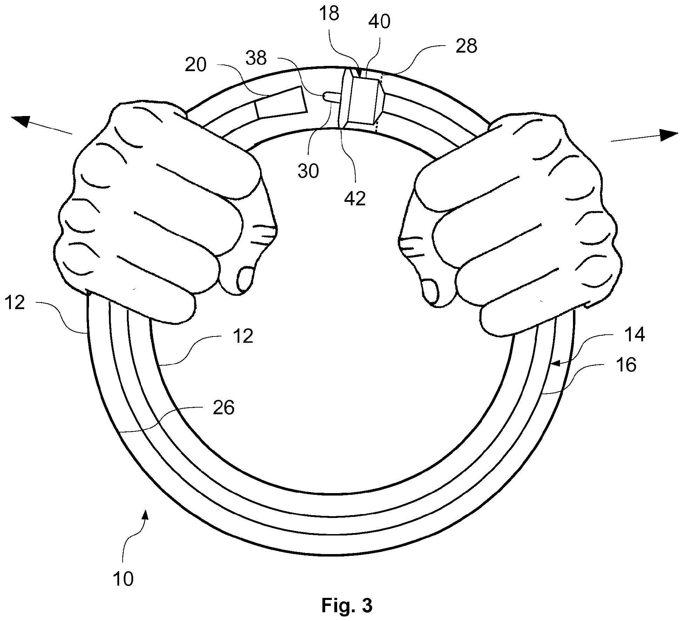

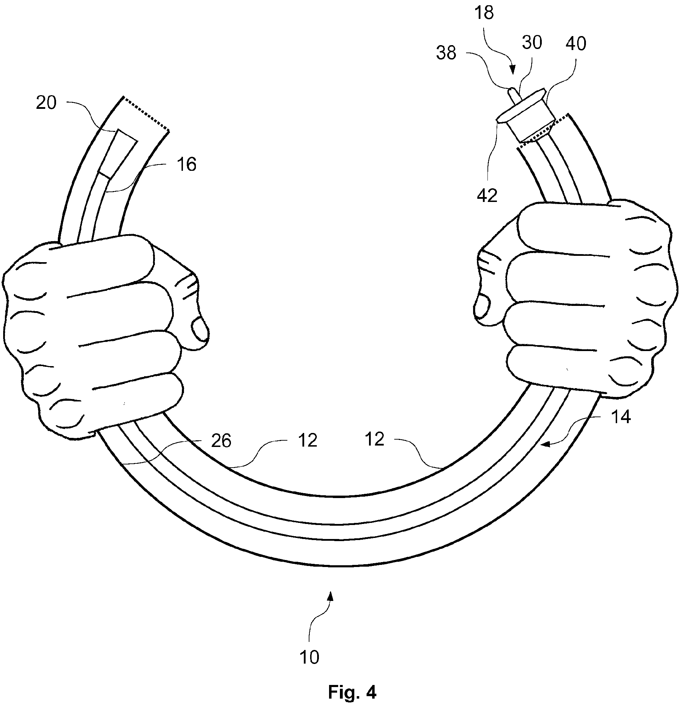

Eine weitere sterile Umverpackung (wie bei bekannten Kathetern üblich) ist erfindungsgemäß nicht erforderlich. In einer bevorzugten Ausführungsform umfasst das Kathetersystem daher neben der schlauchförmigen Verpackung (die sowohl als Sterilbarriere als auch als Reservoir für Gleitmittel wirkt) keine weitere (sterile) Umverpackung. Auf diese Weise wird sichergestellt, dass das Kathetersystem, wie in den Figuren 3 und 4 erkennbar, auch durch motorisch eingeschränkte Patienten einfach zu öffnen ist. Dies schließt selbstverständlich nicht aus, dass das Kathetersystem von weiteren Verpackungen z. B. für Verkaufs- und Transportzwecke, z. B. zur Verpackung mehrerer Kathetersysteme innerhalb eines Pakets, umschlossen sein darf.

Das distale Ende des Katheters bzw. das Einführelement kann an der Innenseite der Verpackung im distalen Endbereich der schlauchförmigen Verpackung angebracht bzw. befestigt sein. Diese Befestigung des Katheters bzw. des Einführelements verhindert ein Verrutschen des Einführelements innerhalb der ringförmig geschlossenen Verpackung und ebenso ein Verrutschen des Katheters bzw. des Einführelements während des Einführens des Kathetertubus in die Harnröhre und die Harnblase. Die Anbringung bzw. Befestigung kann z. B. durch Verkleben oder Verschweißen der Verpackung an oder mit dem distalen Ende des Katheters bzw. dem Einführelement, z. B. an dem Spitzenhalter, bewirkt sein.

Die Verpackung umfasst kann zwischen dem distalen Endbereich und dem proximalen Endbereich der schlauchförmigen Verpackung eine Sollreißstelle.

"Zwischen" betrifft in diesem Zusammenhang jeweils die Seite der Endbereiche, die nach dem Aufreißen der Verpackung offen ist. Mit anderen Worten bezeichnet oder umfasst "zwischen den Endbereichen" den Bereich, an dem das distale und das proximale Ende des Katheters innerhalb der Verpackung aufeinandertreffen bzw. am Dichtesten zusammenliegen bzw. einen Bereich der in geringem Abstand proximal dieses Bereichs, insbesondere proximal des distalen Endes des Katheters, liegt. Das distale Ende des Katheters kann somit nach dem Öffnen der Verpackung aus der Verpackung hinausragen.

Die Sollreißstelle ist geeignet, das Aufreißen der Verpackung an dieser Stelle zu erleichtern und sicherzustellen, dass die ringförmig geschlossene Verpackung bevorzugt an dieser Stelle reißt. Durch dieses Aufreißen der Verpackung wird der Ringschluss der Verpackung aufgebrochen, so dass die schlauchförmige Verpackung in der Folge nicht mehr ringförmig geschlossen ist. Darüber hinaus beeinträchtigt die Sollreißstelle (im geschlossenen Verpackungszustand) nicht die Wirkung der Verpackung als Sterilbarriere. Geeignete Möglichkeiten, eine Sollreißstelle in die Verpackung einzufügen, sind Fachleuten bekannt. So kann die Sollreißstelle z.B. durch Verschweißen oder Verkleben von Seitenbereichen der Verpackung und anschließendes Einbringen einer oder mehrerer linienförmiger, zur Längsrichtung der Verpackung orthogonalen, Perforationen in die verschweißten oder verklebten Seitenbereiche ausgebildet sein. Hierfür kann nur eine Seite der Verpackung auf z. B. 0,5 cm Breite verschweißt oder verklebt sein, oder zwei sich gegenüberliegende Seiten. Alternativ ist es auch denkbar, eine oder mehrere das Verpackungsmaterial nicht vollständig durchdringende Performationen quer zur Längsrichtung in der schlauchförmigen Verpackung auszubilden. So wird die Sterilbarrierenfunktion nicht beeinträchtigt und dennoch ein einfaches Aufreißen der Verpackung gewährleistet.

Die Sollreißstelle liegt bevorzugt proximal des distalen Endes des Einführelements, insbesondere unmittelbar proximal, z. B. zwischen 0,5 und 3 cm proximal des distalen Ende des Einführelements. Auf diese Weise wird sichergestellt, dass nach dem Aufreißen der Verpackung das distale Ende des Katheters freiliegt und ohne Behinderung durch die Verpackung in die Harnröhre eingeführt werden kann.

Bevorzugt wird durch das Aufreißen der Verpackung an der Sollreißstelle das distale Katheterende (unmittelbar) frei zugänglich und kann in eine Harnröhre eingeführt werden, ohne dass weitere Entpackungsschritte notwendig sind. Anders gesagt ist der Katheter unmittelbar nach dem Aufreißen der Verpackung an der Sollreißstelle gebrauchsfertig bzw. einsatzbereit.

In einem weiteren Aspekt betrifft die Erfindung ein Verfahren zur intermittierenden Katheterisierung mittels eines erfindungsgemäßen Kathetersystems bzw. eine Verwendung eines erfindungsgemäßen Kathetersystems zur intermittierenden Katheterisierung, vorzugsweise zur intermittierenden Selbstkatheterisierung, Schritte umfassend, bei denen man: a) die Verpackung an der Sollreißstelle aufreißt; b) optional die Vorlaufspitze des Einführelements in die Harnröhre einführt; c) den Kathetertubus in die Harnröhre und die Harnblase einführt; und d) Urin durch den Kathetertubus (und den Auslass) abführt. Es ist erfindungsgemäß bevorzugt, dass durch das Aufreißen in Schritt a) das distale Katheterende frei zugänglich ist und in die Harnröhre eingeführt werden kann, ohne dass weitere Entpackungsschritte notwendig sind.

In Schritt a) der Verwendung wird die Verpackung an einer Sollreißstelle aufgerissen. Hierzu kann der Benutzer z. B., wie in Figur 3 erkennbar, die ringförmige Verpackung mit beiden Händen derart halten, dass die Sollreißstelle zwischen den Händen liegt, und anschließend beide Hände voneinander weg bewegen (Figur 4), so dass die Verpackung an der Sollreißstelle auseinander reißt. Die weiteren Verfahrensschritte sind dem geübten Nutzer von der Benutzung herkömmlicher ISK Katheter bekannt.

Das erfindungsgemäße Kathetersystem und Verfahren haben den Vorteil und sind dadurch gekennzeichnet, dass der Katheter unmittelbar nach dem Aufreißen der Verpackung an der Sollreißstelle einsatzbereit ist, d. h. in die Harnröhre eingeführt werden kann. Insbesondere ist es nicht erforderlich, weitere Verpackungsteile oder andere Umhüllungen zu öffnen und hierdurch die Sterilität des Katheters zu gefährden. Insbesondere bedeutet dies, dass das erfindungsgemäße Kathetersystem und Verfahren dadurch gekennzeichnet sind, dass das zur Einführung in die Harnröhre vorgesehene distale Ende des Katheters (z. B. das distale (atraumatische) Ende des Katheters, das distale, atraumatische Ende des Kathetertubus oder das distale Ende des Einführelements) unmittelbar nach dem Aufreißen der Verpackung an der Sollreißstelle frei zugänglich ist (d. h. nicht von einer weiteren Verpackung bedeckt ist) und in die Harnröhre eingeführt werden kann, ohne dass weitere Entpackungsschritte notwendig sind. Dies schließt selbstverständlich nicht aus, dass sich z. B. während des Einführungsvorgangs die Verpackung vom proximalen Endbereich des Katheters her über dem Kathetertubus zusammenschieben darf.

### Kurze Beschreibung der Figuren

In den Zeichnungen sind Ausführungsbeispiele der Erfindung schematisch dargestellt. Es zeigen:
- **Fig. 1**: eine schematische Darstellung eines erfindungsgemäßen Kathetersystems;
- **Fig. 2**: eine schematische Darstellung einer weiteren Ausführungsform eines erfindungsgemäßen Kathetersystems, die Auslass und Einführelement umfasst;
- **Fig. 3**: das in Fig. 2 dargestellte Kathetersystem mit Händen zur Veranschaulichung des einfachen Aufreißmechanismus;
- **Fig. 4**: das in Figs. 2 und 3 dargestellte Kathetersystem mit Händen zur Veranschaulichung des einfachen Aufreißmechanismus nach dem Aufreißen der Verpackung an der Sollreißstelle; und
- **Fig. 5**: das in Figs. 2-4 dargestellte, aufgerissene Kathetersystem und einen Penis, wobei die Vorlaufspitze und die Harnröhrenöffnung aufeinander ausgerichtet sind.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

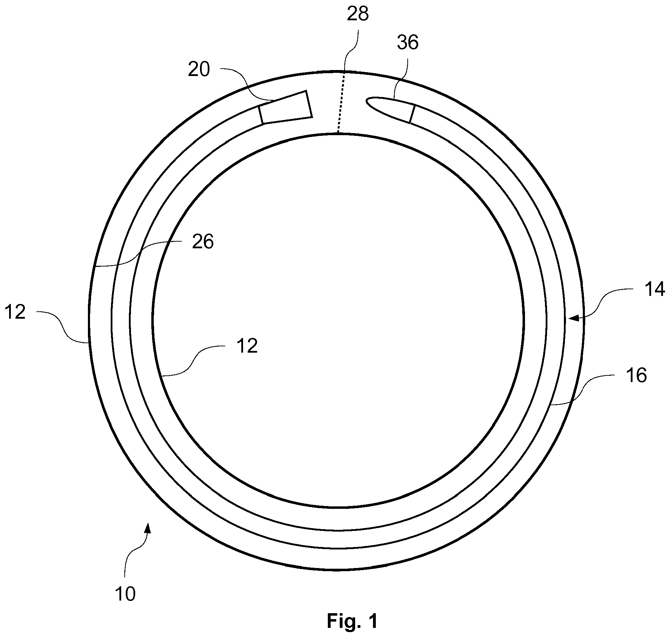

Fig. 1 zeigt ein erfindungsgemäßes Kathetersystem 10 in einer schematischen Darstellung. Das Kathetersystem 10 umfasst eine schlauchförmige Verpackung 12, die ringförmig geschlossen ist, und einen Katheter 14, der für die intermittierende (Selbst-) Katheterisierung geeignet ist. Die schlauchförmige Verpackung 12 ist aus einem flexiblen Kunststoffmaterial, das als Sterilbarriere wirkt.

Der Katheter 14 umfasst einen trichterförmigen Auslass 20, einen Kathetertubus 16, der etwa 41 cm lang ist, und einen distalen Endbereich 36, der das Einführen des Kathetertubus in die Harnröhre und die Harnblase unterstützt und atraumatisch ausgebildet ist.

Der Auslass 20 und der distale Endbereich 36 des Katheters 14 liegen in der Verpackung 12 voreinander und sind voneinander beabstandet. Alternativ könnte das distale Ende des Katheters 14 in den Auslass 20 eingeschoben sein. Darüber hinaus könnte das distale Ende des Katheters 14 auch mechanisch, z. B. durch einen Verriegelungsmechanismus, mit dem Auslass 20, z. B. mit dem Inneren des Auslasses 20, verbunden sein. Zwischen dem Auslass 20 und dem distalen Ende 36 des Katheters angeordnet, umfasst die Verpackung 12 eine Sollreißstelle 28. An der Sollreißstelle 28 wird die Verpackung aufgerissen bzw. aufreißen, wenn an beiden Seiten von der Sollreißstelle 28 an dem Kathetersystem 10 gezogen wird. Die Sollreißstelle 28 ist als eine nicht das Verpackungsmaterial durchdringende Perforation ausgebildet, die um die Verpackung herum umläuft und die Sterilität im Verpackungsinneren nicht gefährdet. Dies bedeutet, dass die Perforation die Funktion der Sterilbarriere nicht beeinträchtigt.

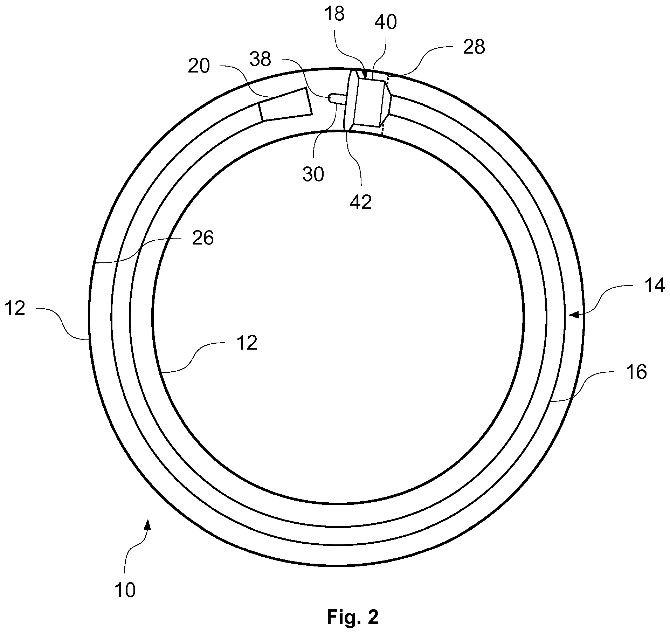

In Fig. 2 ist eine weitere Ausführungsform des erfindungsgemäßen Kathetersystems 10 dargestellt. Die in Fig. 2 dargestellte Ausführungsform unterscheidet sich von der in Fig. 1 dargestellten Ausführungsform darin, dass sie am distalen Ende des Katheters 14 ein Einführelement 18 aufweist.

Das Einführelement 18 weist einen Spitzenhalter 40 und weiter distal einen Kragen 42 auf, der umlaufend im distalen Endbereich des Einführelements 18 ausgebildet ist. Weiter distal weist das Einführelement 18 eine Vorlaufspitze 30 auf, die zur Einführung in die Harnröhre geeignet ist. Die Vorlaufspitze 30 weist an ihrem distalen Ende eine abgerundete Form auf, um das Verletzungsrisiko zu reduzieren. Darüber hinaus ist das distale Ende der Vorlaufspitze 30 mit einer Tubusdurchführung 38 versehen, durch die der Kathetertubus 16 hindurchgeführt und in die Harnröhre und die Harnblase vorgeschoben werden kann.

Der Auslass 20 und das Einführelement 18 des Katheters 14 liegen in der Verpackung 12 voreinander und sind voneinander beabstandet. Alternativ könnte die Vorlaufspitze 30 des Einführelements 18 des Katheters 14 in den Auslass 20 eingeschoben sein.

Über dem Spitzenhalter 40 des Einführelements 18 angeordnet, umfasst die Verpackung 12 eine Sollreißstelle 28. An der Sollreißstelle 28 wird die Verpackung aufreißen, wenn an beiden Seiten von der Sollreißstelle 28 an dem Kathetersystem 10 gezogen wird. Die Sollreißstelle 28 ist als nicht das Verpackungsmaterial durchdringende Perforation ausgebildet, die um die Verpackung herum umläuft und die Sterilität im Verpackungsinneren nicht gefährdet. Dies bedeutet, dass die Perforation ebenfalls die Funktion einer Sterilbarriere aufweist.

Proximal hinter der Vorlaufspitze 30 und der Sollreißstelle 28 kann die Verpackung 12 in einer nicht dargestellten Weise mit dem Einführelement 18, vorzugsweise mit dem Spitzenhalter, verbunden, z. B. verklebt oder verschweißt, sein.

Fig. 3 zeigt das Kathetersystem aus Fig. 2. Die Darstellung unterscheidet sich lediglich dadurch von Fig. 2, dass hier die Hände eines motorisch eingeschränkten Patienten dargestellt sind, um das Aufreißen der schlauchförmigen Verpackung 12 zu veranschaulichen. Der Patient ist z. B. nicht in der Lage feinmotorische Handlungen mit einzelnen Fingern durchzuführen, kann aber mit der ganzen Hand zugreifen. Es ist erkennbar, dass das Kathetersystem 10 derart in beiden Händen gehalten wird, dass die schlauchförmige Verpackung 12 in den Handinnenflächen der Fäuste liegt und die Sollreißstelle 28 zwischen den beiden Händen liegt. Durch Krafteinwirkung in Richtung der in Fig. 3 dargestellten Pfeile kann der Patient nun die schlauchförmige Verpackung an der Sollreißstelle 28 aufreißen.

Fig. 4 zeigt das Kathetersystem 10 aus Fig. 3 unmittelbar nach dem Aufreißen der Verpackung 12 an der Sollreißstelle 28. Um ein Verrutschen des Einführelements 18 gegenüber der Verpackung 12 zu verhindern, ist die Verpackung proximal hinter dem Kragen 42 auf nicht dargestellte Weise an dem Spitzenhalter 40 befestigt. Es ist erkennbar, dass das Einführelement 18 aus der geöffneten Verpackung 12 herausragt, so dass das Einführen der Vorlaufspitze 30 in die Harnröhre nicht von der Verpackung 12 behindert wird. Der Auslass 20 ist gegenüber dem proximalen Verpackungsende leicht nach innen versetzt. Dies schränkt jedoch die Abführleistung des Auslasses 20 nicht ein. In einer Ausführungsform kann die schlauchförmige Verpackung 12 an ihrem proximalen Verpackungsende vom Anwender so weit in distale Richtung entlang des Kathetertubus 16 verschoben werden, dass der Auslass 20 nicht mehr von der schlauchförmigen Verpackung 12 umschlossen wird und der Auslass 20 z. B. an einen Urinbeutel angeschlossen werden kann.

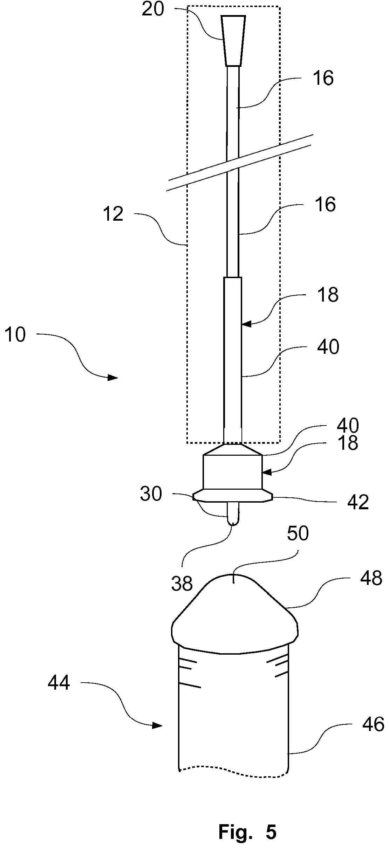

Fig. 5 zeigt ein Verfahren zur Anwendung eines erfindungsgemäßen Kathetersystems 10, insbesondere des in Fig. 4 dargestellten geöffneten Kathetersystems 10. Es ist erkennbar, dass die Vorlaufspitze 30 auf die Harnröhrenöffnung 50 des Penis 44 ausgerichtet ist, so dass die Vorlaufspitze 30 in die Harnröhre eingeführt werden kann. Aufgrund der besseren Darstellbarkeit und insbesondere Sichtbarkeit der einzelnen Elemente ist keine menschliche Hand dargestellt, die den Penis 44 und das Kathetersystem 10 hält.

Der Katheter 14 weist einen Kathetertubus 16 auf, der durch ein Einführelement 18 verläuft. Innerhalb des Einführelements 18 verläuft der Kathetertubus 16 bis kurz vor der Tubusdurchführung 38 und kann nach Einführung der Vorlaufspitze 30 in die Harnröhre einfach durch die Tubusdurchführung 38 hindurchgeschoben werden. Der Katheter weist ferner einen Auslass 20 auf.

Der Kathetertubus 16 verläuft innerhalb der schlauchförmigen Verpackung 12, die ein Gleitgel enthält. Die Verpackung 12 kann durch eine Schweißnaht proximal des Kragens 42 durch eine Schweißnaht an dem Spitzenhalter angebracht sein. Es ist erkennbar, dass die Verpackung 12 und der Katheter 14 nach der Öffnung des Ringschlusses ihre Ringform aufgegeben bzw. verloren haben und im Wesentlichen in gerader Form gehandhabt werden können.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

### Bezugszeichenliste

| | | | |
|---|---|---|---|
| 10 | Kathetersystem | 48 | Eichel |
| 12 | schlauchförmige Verpackung | 50 | Harnröhrenöffnung |
| 14 | Katheter | | |
| 16 | Kathetertubus | | |
| 18 | Einführelement | | |
| 20 | Auslass | | |
| 22 | distaler Endbereich Verpackung | | |
| 24 | proximaler Endbereich Verpackung | | |
| 26 | Innenseite Verpackung | | |
| 28 | Sollreißstelle | | |
| 30 | Vorlaufspitze | | |
| 32 | Harnröhre | | |
| 34 | Harnblase | | |
| 36 | distaler Endbereich Kathetertubus | | |
| 38 | Tubusdurchführung | | |
| 40 | Spitzenhalter | | |
| 42 | Kragen | | |
| 44 | Penis | | |
| 46 | Penisschaft | | |

## Patentansprüche

1. Kathetersystem (10) zur intermittierenden Katheterisierung, umfassend eine schlauchförmige Verpackung (12) und einen die Verpackung (12) durchlaufenden Katheter (14), wobei die schlauchförmige Verpackung (12) ringförmig geschlossen ist, wobei das distale und das proximale Ende der Verpackung (12) im geschlossenen Zustand der Verpackung (12) miteinander verbunden sind und die schlauchförmige Verpackung (12) zwischen ihrem distalen Endbereich (22) und ihrem proximalen Endbereich (24) eine Sollreißstelle (28) umfasst.

2. Kathetersystem (10) nach Anspruch 1, **dadurch gekennzeichnet, dass** der Ringschluss durch Verschweißen oder Verkleben des distalen Endbereichs (22) mit dem proximalen Endbereich (24) der Verpackung (12) hergestellt ist.

3. Kathetersystem (10) nach einem der vorangegangenen Ansprüche, **dadurch gekennzeichnet, dass** die Verpackung (12) eine Sterilbarriere ist.

4. Kathetersystem (10) nach einem der vorangegangenen Ansprüche, **dadurch gekennzeichnet, dass** durch das Aufreißen der Verpackung (12) an der Sollreißstelle (28) das distale Katheterende frei zugänglich wird und in eine Harnröhre eingeführt werden kann, ohne dass weitere Entpackungsschritte notwendig sind.

5. Kathetersystem (10) nach einem der vorangegangenen Ansprüche, **dadurch gekennzeichnet, dass** der Katheter (14) einen Kathetertubus (16), ein Einführelement (18) an seinem distalen Ende und einen Auslass (20) an seinem proximalen Ende umfasst.

6. Kathetersystem (10) nach Anspruch 5, **dadurch gekennzeichnet, dass** das Einführelement (18) in der Verpackung (12) neben, vor oder innerhalb des Auslasses (20) liegt.

7. Kathetersystem (10) nach einem der Ansprüche 5 oder 6, **dadurch gekennzeichnet, dass** der Auslass (20) trichterförmig ausgebildet ist.

8. Kathetersystem (10) nach einem der Ansprüche 5-7, **dadurch gekennzeichnet, dass** angrenzend zwischen der Innenseite (26) der Verpackung (12) und dem Kathetertubus (16) ein Gleitmittel eingebracht ist.

9. Kathetersystem (10) nach einem der Ansprüche 5-8, **dadurch gekennzeichnet, dass** das Einführelement (18) an der Innenseite (26) der Verpackung (12) im distalen Endbereich (22) der schlauchförmigen Verpackung (12) angebracht ist.

## Claims

1. Catheter system (1) for intermittent catheterisation, comprising a tube-shaped package (12) and a catheter (14) extending through the package (12), wherein
the tube-shaped package (12) is annularly closed, wherein, in the closed stated of the package (12), the distal and proximal end of the package (12) are connected to each other, and between its distal end area (22) and its proximal end area (24), the package (12) has a predetermined tearing point (28).

2. Catheter system (10) according to claim 1, **characterised in that** the annular closure is produced through welding or adhering the distal end area (22) to the proximal end area (24) of the package (12).

3. Catheter system (10) according to any one of the preceding claims, **characterised in that** the package (12) is a sterile barrier.

4. Catheter system (10) according to any one of the preceding claims, **characterised in that** through tearing open the package (12) at the predetermined tearing point (28), the distal catheter end becomes freely accessible and can be introduced into a urethra without further unpackaging steps being necessary.

5. Catheter system (10) according to any one of the preceding claims, **characterised in that** the catheter (14) comprises a catheter tube (16), an introduction element (18) at its distal end and an outlet (20) at its proximal end.

6. Catheter system (10) according to claim 5, **characterised in that** the introduction element (18) lies in the package (12) next to, in front of or inside the outlet (20).

7. Catheter system (10) according to any one of claims 5 or 6, **characterised in that** the outlet (20) is funnel-shaped in design.

8. Catheter system (10) according to any of claims 5 to 7, **characterised in that** adjacently between the inside (26) of the package (12) and the catheter tube (16), a lubricant is introduced.

9. Catheter system (10) according to any one of claims 5 to 8, **characterised in that** the introduction element (18) is applied on the inside (26) of the package (12) in the distal end area (22) of the tube-shaped package (12) .

## Revendications

1. Système de cathéter (10) pour cathétérisme intermittent comprenant un emballage tubulaire (12) et un cathéter (14) logé dans l'emballage (12), l'emballage tubulaire (12) étant fermé en forme d'anneau, l'extrémité distale et l'extrémité proximale de l'emballage (12) étant jointes lorsque l'emballage (12) est fermé et l'emballage tubulaire (12) présentant une ligne de rupture de consigne (28) entre sa section d'extrémité distale (22) et sa section d'extrémité proximale (24).

2. Système de cathéter (10) selon la revendication 1, **caractérisé en ce que** la jonction annulaire est réalisée par soudage ou collage de la section d'extrémité distale (22) et de la section d'extrémité proximale (24) de l'emballage (12).

3. Système de cathéter (10) selon l'une des revendications précédentes, **caractérisé en ce que** l'emballage (12) est une barrière stérile.

4. Système de cathéter (10) selon l'une des revendications précédentes, **caractérisé en ce que** le déchirement de l'emballage (12) à la ligne de rupture de consigne (28) libère l'extrémité distale du cathéter qui devient librement accessible et peut être introduite dans un urètre sans nécessiter d'autres étapes de déballage.

5. Système de cathéter (10) selon l'une des revendications précédentes, **caractérisé en ce que** le cathéter (14) comprend un tube de cathéter (16), un élément d'introduction (18) à son extrémité distale et un orifice de sortie (20) à son extrémité proximale.

6. Système de cathéter (10) selon la revendication 5, **caractérisé en ce que,** dans l'emballage (12), l'élément d'introduction (18) est posé à côté, devant ou à l'intérieur de l'orifice de sortie (20)

7. Système de cathéter (10) selon l'une des revendications 5 ou 6, **caractérisé en ce que** l'orifice de sortie (20) est réalisé en forme d'entonnoir.

8. Système de cathéter (10) selon l'une des revendications 5 à 7, **caractérisé en ce que** un lubrifiant est introduit entre la face interne (26) de l'emballage (12) et le tube de cathéter (16).

9. Système de cathéter (10) selon l'une des revendications 5 à 8, **caractérisé en ce que** l'élément d'introduction (18) est agencé contre la face interne (26) de l'emballage tubulaire (12) dans la section d'extrémité distale (22) de celui-ci.
